# EUROPÄISCHE PATENTSCHRIFT

(11) **EP 2 809 267 B1**
(45) Veröffentlichungstag und Bekanntmachung des Hinweises auf die Patenterteilung: **28.12.2016**
(21) Anmeldenummer: 13705933.3
(22) Anmeldetag: 01.02.2013
(51) Int. Cl.: A61F 2/07

(54) **INTRALUMINALE GEFÄßPROTHESE**
INTRALUMINAL VASCULAR PROSTHESIS
PROTHÈSE VASCULAIRE ENDOLUMINALE

(30) Priorität: 01.02.2012 DE 102012100839
(43) Veröffentlichungstag der Anmeldung: 10.12.2014
(73) Patentinhaber: JOTEC GmbH, 72379 Hechingen (DE)
(72) Erfinder: BOGENSCHUETZ, Thomas, 72379 Hechingen-Stein (DE); BARTHOLD, Franz-Peter, 72336 Balingen (DE)
(74) Vertreter: Witte, Weller & Partner Patentanwälte mbB
(86) Internationale Anmeldenummer: PCT/EP2013/052037
(87) Internationale Veröffentlichungsnummer: WO 2013/113876

(56) Entgegenhaltungen:
- WO-A1-2005/058202
- WO-A1-2008/130572
- US-A- 6 152 956
- US-A1- 2006 276 883

## Beschreibung

Die vorliegende Erfindung betrifft eine intraluminale Gefäßprothese, insbesondere zur Implantation in den Aortenbogen, die einen hohlzylindrischen Körper mit einem ersten Ende und einem zweite Ende aufweist, wobei die Gefäßprothese an ihrem ersten Ende einen ersten Gefäßprothesenabschnitt mit hintereinander angeordneten Ringen aus mäanderförmig umlaufenden Stützen und ein an den Ringen befestigtes und diese verbindendes Prothesenmaterial umfasst.

Insbesondere betrifft die vorliegende Erfindung Gefäßprothesen, die im Bereich des Aortenbogens implantiert werden.

Allgemein ist es bekannt, intraluminale Gefäßprothesen, die auch als endovaskuläre Gefäßstents/-stentgrafts bezeichnet werden, zur Behandlung von Aneurysmen in Arterien zu implantieren. Unter einem Aneurysma versteht man dabei eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Aneurysmen treten häufig im Bereich der Baucharterie (Aorta abdominalis) oder Brustarterie (Aorta thoracica) auf. Zur Behandlung von Aneurysmen in der Bauch- oder Brustarterie ist es bereits bekannt, die Arterie durch Implantation eines Stents soweit zu stabilisieren, dass eine Ruptur des Gefäßes vermieden wird.

Die zur Behandlung derartiger Aneurysmen verwendeten Gefäßprothesen oder -implantate bestehen im Allgemeinen aus einem hohlzylindrischen Metallrahmen, dessen Mantelfläche mit einer Textil- oder Polymerfolie, also einem Prothesenmaterial, abgedeckt ist, so dass sich ein hohlzylindrischer Körper ergibt. Zur Implantation wird die Gefäßprothese radial zusammengedrückt, so dass sich seine Querschnittsfläche deutlich verringert. Die Gefäßprothese wird dann mit Hilfe eines Einführsystems in den Bereich des Aneurysma gebracht, wo sie freigesetzt und ggf. vernäht wird. Auf Grund der Federwirkung des Metallrahmens expandiert die Gefäßprothese wieder in ihre ursprüngliche Form und spannt dabei ihre Mantelfläche auf, die sich proximal und distal von dem Aneurysma innen in dem Blutgefäß verklemmt. Auf diese Weise fließt das Blut jetzt durch die Gefäßprothese und eine weitere Belastung der Aussackung wird verhindert. Der Metallrahmen der Gefäßprothese, der von dem Prothesenmaterial bedeckt ist, besteht dabei bspw. aus hintereinander angeordneten sogenannten Stentringen aus einem selbstexpandierenden Material. Eine Gefäßprothese ist in WO2005058202 A1 beschrieben. Die Gefäßprothese besteht aus zwei Stent Grafts, die mit einer Brücke bestehend aus einem Stent miteinander verbunden sind.

Ein Aneurysma kann nicht nur in der Baucharterie oder Brustarterie auftreten, sondern auch in dem sogenannten aufsteigenden Ast der Aorta (Aorta ascendens). Der aufsteigende Ast der Aorta ist unmittelbar mit dem Herz verbunden. Ausgehend von der Aortenwurzel (Sinus aortae) verläuft der aufsteigende Ast in leicht gekrümmter Form vom Herz weg nach oben und geht dort in den Aortenbogen (Arcus aortae) über. Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit der Brustarterie und im weiteren Verlauf mit der Baucharterie.

Ein Aneurysma oder eine Dissektion im aufsteigenden Ast der Aorta wird bis heute in einem invasiven offenen Eingriff behandelt. Eine solche Operation macht bisher regelmäßig zwei große, zeitlich getrennte Eingriffe erforderlich, und stellt einen sehr großen und aufwändigen und damit gefährlichen Eingriff dar, da nicht nur das Herz, sondern auch das Gehirn und die Bauchorgane des Patienten einer hypothermen Perfusion, d.h. also einer künstlichen, kalten extrakorporalen Durchblutung, bzw. einem hypothermen Durchblutungsstillstand unterzogen werden müssen. Mit einem solchen Eingriff sind jedoch nur wenige Herzchirurgen an erfahrenen Zentren vertraut.

Bisher ist im Stand der Technik keine Gefäßprothese oder Stent-/Stetngraftsystem bekannt, mit Hilfe dessen der oben geschilderte Eingriff vereinfacht und zeitlich verkürzt werden könnte, weshalb nach wie vor ein großes Bedürfnis nach einem solchen System besteht.

Aufgabe der vorliegenden Erfindung ist es daher, ein System bereitzustellen, mit welchem der Bereich der aufsteigenden Aorta, des Aortenbogens und der absteigendem Aorta schnell und unkompliziert behandelt werden kann, bzw. das die oben geschilderten Eingriffe auch weniger erfahrenen Herzchirurgen ermöglicht.

Diese Aufgabe wird durch eine intraluminale Gefäßprothese gelöst, die einen hohlzylindrischen Körper mit einem ersten Ende und einem zweite Ende aufweist, wobei die Gefäßprothese an ihrem ersten Ende einen ersten Gefäßprothesenabschnitt mit hintereinander angeordneten Ringen aus mäanderförmig umlaufenden Stützen und ein an den Ringen befestigtes und diese verbindendes Prothesenmaterial umfasst, und wobei die Gefäßprothese an ihrem zweiten Ende einen zweiten Gefäßprothesenabschnitt aufweist, welcher leidglich ein Prothesenmaterial und keine damit verbundenen, hintereinander angeordnete Ringe aus mäanderförmig umlaufenden Stützen aufweist, wobei die Gefäßprothese ferner einen zwischen dem ersten und dem zweiten Gefäßprothesenabschnitt vorliegenden und mit diesen Gefäßprothesenabschnitten fest verbundenen Stentabschnitt aufweist, der von Prothesenmaterial frei ist.

Durch die erfindungsgemäße Kombination aus zwei relativ kurzen Gefäßprothesenabschnitten, einem ersten, der einen mit Prothesenmaterial bedeckten - sogenannten "gecoverten" - Gefäßprothesenabschnitt darstellt, und einem zweiten, der lediglich aus Prothesenmaterial besteht, mit einem zwischen diesen Gefäßprothesenabschnitten liegenden, von Prothesenmaterial freien - sogenannten "nicht-gecoverten" - Stentabschnitt wird es möglich, die drei Abschnitte der Aorta, also die Aorta ascendens (aufsteigenden Hauptschlagader), des Aortenbogens und der descendierenden Aorta (absteigenden Hauptschlagader) simultan zu behandeln, und gleichzeitig den Zeitaufwand des chirurgischen Eingriffs signifikant zu senken. Damit kann auf die Resektion des Aortenbogens mit all den damit verbundenen komplexen Perfusionsanforderungen für Gehirn und unteren Körper verzichtet werden, und der Eingriff nur mit Durchtrennung des obersten Abschnitts der aufsteigenden Aorta in einer kurzen, ca. 10 -20 minütigen selektiven Kopfperfusionsphase oder hypothermen Stillstandsphase erfolgen, um die neue Gefäßprothese einzuführen und freizusetzen. Die Einführung und Freisetzung kann dabei einfach unter Sicht des Auges bzw. eines Angioskops erfolgen.

Erfindungsgemäß wird damit erstmals eine intraluminale Gefäßprothese bereitgestellt, mit welcher die Möglichkeit geschaffen wird, Eingriffe am Aortenbogen, bzw. in der aufsteigenden Aorta, dem Aortenbogen und der absteigenden Aorta, chirurgisch zu vereinfachen und zeitlich deutlich zu verkürzen. Vorteilhafterweise können damit nicht nur hoch spezialisierte Herzchirurgen die weiter oben geschilderten Eingriffe am Aortenbogen vornehmen. Ferner kann die erfindungsgemäße Gefäßprothese auch bei schwer erkrankten Patienten und bei älteren Patienten mit bspw. altersbedingt beschädigten Aortenwandschichten und mit Nicht-Durchblutung von lebenswichtigen Organsystemen wie Gehirn oder Bauchorganen eingesetzt werden, da diese mit einem zeitlich verkürzten und chirurgisch vereinfachten Verfahren eingesetzt werden können.

Die drei Abschnitte der Gefäßprothese bilden eine Einheit und können aus einem Stückgefertigt sein, bzw. sind fest miteinander, bspw. durch Vernähen, verbunden.

Vorteilhafterweise ist dabei der von Prothesenmaterial freie Stentabschnitt im expandierten Zustand im Bereich des Aortenbogens freisetzbar. Dadurch wird gewährleistet, dass der Blutfluss in die abgehenden Gefäße, wie den Truncus brachiocephalicus, die linke Aorta carotis communis, und die linke Arteria subclavia, nicht beeinträchtigt wird. Das durch den Aortenbogen und die hierin zu verankernde Gefäßprothese fließende Blut kann diese durch die im Stentabschnitt vorliegenden Öffnungen der Gefäßprothese verlassen; gleichzeitig ist sicher gestellt, dass die Gefäßprothese über die links und rechts vom Stentabschnitt liegenden Gefäßprothesenabschnitte sicher im Gefäß verankert ist; dies kann einerseits über radiale Expansionskräfte des ersten Gefäßprothesenabschnitts bzw. der darin vorliegenden Ringe und das dadurch bewirkte Anliegen der Gefäßprothese in diesen Bereichen an die Gefäßwand erzielt werden, sowie durch ein Vernähen des zweiten Gefäßprothesenabschnitts, der erfindungsgemäß keine - bzw. nur optional - mäanderförmig umlaufenden Ringe und Stützen aufweist, mit der Aortenwand.

Daher ist gemäß einer weiteren Ausführungsform der erfindungsgemäßen intraluminalen Gefäßprothese bevorzugt, dass zumindest der erste Gefäßprothesenabschnitt bzw. vielmehr dessen Ringe und der von Prothesenmaterial freie Stentabschnitt aus einem selbst-expandierenden Material sind oder ein solches aufweisen. Selbstexpandierbare Materialien sind dem Fachmann im Gebiet der Gefäßimplantate bekannt, wobei hier bspw. Nitinol als bevorzugtes Material aufgeführt wird.

Das bei der erfindungsgemäßen Gefäßprothese verwendete Prothesenmaterial, das in dem ersten und zweiten Gefäßprothesenabschnitt vorgesehen ist, ist dabei vorzugsweise ausgewählt aus Polyester, Polyurethan, Polytetrafluoräthylen oder ultrahochmolekulargewichtiges Polyethylen (UHMPE) und stellt vorzugsweise ein Polyestergewebe dar.

Gemäß einer weiteren bevorzugten Ausführungsform weist der erste Gefäßprothesenabschnitt zwischen zwei, drei, vier und fünf, vorzugsweise drei, hintereinander angeordnete Ringe aus mäanderförmig umlaufenden Stützen auf. Diese Ringe sind dabei lediglich über das Prothesenmaterial miteinander verbunden. Die Stützen bzw. Ringe selber sind untereinander nicht durch Stege o.ä. miteinander verbunden, bzw. stehen nicht in unmittelbaren Kontakt miteinander. Diese Ausführungsform hat den Vorteil, dass der Fachmann die Länge des Gefäßprothesenabschnitts den jeweiligen Gefäßgegebenheiten, also insbesondere dem Durchmesser des Gefäßes und der gewünschten Länge im Gefäß, anpassen kann.

Bei der erfindungsgemäßen Gefäßprothese ist vorgesehen, wenn der erste "gecoverte" Gefäßprothesenabschnitt distal der Arteria subclavia zu liegen kommt, der "nicht-gecoverte" Stentabschnitt im Aortenbogen und der zweite Gefäßprothesenabschnitt im proximalen Aortenbogen.

Mit "proximal" wird dabei die Richtung bzw. Lage oder Position bezeichnet, die näher zum Herzen hinführt, wohingegen mit "distal" die Richtung bzw. Lage oder Position bezeichnet wird, die weiter vom Herzen entfernt liegt bzw. zu liegen kommt oder weg führt.

Anders ausgedrückt befindet sich daher der erste Gefäßprothesenabschnitt am distalen Endbereich der Gefäßprothese und kann daher auch als distaler Gefäßprothesenabschnitt bezeichnet werden, und der zweite Gefäßprothesenabschnitt befindet sich am proximalen Endbereich der Gefäßprothese, der daher als proximaler Gefäßprothesenabschnitt bezeichnet werden kann. Der nicht-gecoverte Stentabschnitt stellt daher anders ausgedrückt den dritten Gefäßprothesenabschnitt dar, der einen nicht-gecoverten Stentabschnitt der Gefäßprothese darstellt.

Der erste "gecoverte" und der zweite Gefäßprothesenabschnitt sowie der dazwischen liegende "nicht-gecoverte" Stentabschnitt weisen allgemein jeweils ein proximales und ein distales Ende auf; insgesamt bilden dabei das distale Ende des ersten Gefäßprothesenabschnitts und das proximale Ende des zweiten Gefäßprothesenabschnitts die äußersten Enden der Gefäßprothese, wobei der erste Gefäßprothesenabschnitt am distalen Ende der Gefäßprothese vorgesehen ist, und der zweite Gefäßprothesenabschnitt am proximalen Ende. Dabei ist das distale Ende des zweiten Gefäßprothesenabschnitts mit dem proximalen Ende des "nicht-gecoverten" Stentabschnitts verbunden, und das distale Ende des "nicht-gecoverten" Stentabschnitts mit dem proximalen Ende des ersten Gefäßprothesenabschnitts.

Gemäß einer weiteren Ausführungsform ist auch bevorzugt, wenn der zweite Gefäßprothesenabschnitt zwischen null und fünf hintereinander angeordnete Ringe aus mäanderförmig umlaufenden Stützen aufweist. Der zweite Gefäßprothesenabschnitt, der im proximalen Aortenbogen zu liegen kommt, kann in diesem Bereich der Aorta angenäht werden, so dass dieser Stentgraftabschnitt nicht zwingend umlaufende Ringe/Stützen aufweisen muss.

In einer bevorzugten Ausführungsform der erfindungsgemäßen Gefäßprothese weist der erste Gefäßprothesenabschnitt drei hintereinander angeordnete Ringe aus mäanderförmig umlaufenden Stützen auf, die über das Prothesenmaterial miteinander verbunden sind, und der zweite Gefäßprothesenabschnitt besteht lediglich aus Prothesenmaterial und weist keine Ringe oder Stützen auf, die über das Prothesenmaterial indirekt miteinander verbunden sind.

Der von Prothesenmaterial freie Stentabschnitt ist gemäß einer Ausführungsform ein geflochtenes oder gezwirbeltes Drahtgeflecht bzw. weist ein solches Drahtgeflecht auf. Dabei wird unter "Drahtgeflecht" jede Ausbildung eines Stents verstanden, bei der verschiedene Draht-Stränge derart miteinander verflochten, ineinander verschlungen oder anderweitig verknüpft sind, dass sich eine Struktur mit Zonen, Bereichen oder Punkten bildet, an denen die Stränge übereinander liegen, und mit Zonen oder Bereichen, die frei von den Drahtsträngen sind und daher Öffnungen oder Fenster oder Maschen bilden.

Gemäß einer alternativen Ausführungsform ist der von Prothesenmaterial freie Stentabschnitt ein Laser-geschnittenes Rohr. Auch diese Ausführungsform weist Maschen oder Öffnungen auf, über die das in der Aorta bzw. im Aortenbogen geführte Blut die Aorta in die abgehenden Gefäße, insbesondere der Truncus brachiocephalicus, die linke Aorta carotis communis, und die linke Arteria subclavia, verlassen kann und damit eine Durchblutung dieser Gefäße gewährleistet.

Gemäß einer besonders bevorzugten Ausführungsform weisen der erste und der zweite Gefäßprothesenabschnitt eine Länge von zwischen ca. 20 mm und ca.100 mm auf, sowie der nicht-gecoverte Stentabschnitt eine Länge von zwischen ca. 30 mm und ca. 100 mm.

Ferner wird ein beispielhaftes Verfahren zur Freisetzung der erfindungsgemäßen intraluminalen Gefäßprothese beschrieben, wobei das Verfahren die folgenden Schritte aufweist:
- Einbringen der intraluminalen Gefäßprothese in die Aorta im komprimierten Zustand, derart, dass der erste Gefäßprothesenabschnitt vollständig distal der Arteria subclavia positioniert wird;
- Überführen der intraluminalen Gefäßprothese in den expandierten Zustand derart, dass der von Prothesenmaterial freie Stentabschnitt im Bereich der Abgänge des Truncus brachiocephalus, der Arteria carotis communis, und der Arteria subclavia sinistra im Aortenbogen freigesetzt wird, und der zweite Gefäßprothesenabschnitt proximal des Abgangs des Truncus brachiocephalus freigesetzt wird.

Mit diesem Verfahren wird sicher gestellt, dass die erfindungsgemäße Gefäßprothese derart positioniert wird, dass der nicht-gecoverte Stentabschnitt den Blutfluss in die abgehenden Gefäße Truncus brachiocephalus, Arteria carotis communis, und Arteria subclavia sinistra ermöglicht.

Hierbei wird darauf geachtet, dass der erste gecoverte Gefäßprothesenabschnitt mit seinem proximalen Ende - das mit dem distalen Ende des nicht-gecoverten Stentabschnitts verbunden ist - knapp distal des Arteria subclavia Abgangs endet. Der nicht gecoverte Stentabschnitt wird nun im Aortenbogen freigesetzt, wobei die Drahtmaschen bzw. die Öffnungen des Laser-geschnittenen Stentabschnitts so weiträumig sind, dass keine Gefährdung im Sinn einer Verlegung der Abgänge der Kopf-Hals Gefäße (Truncuns brachiocephalicus, linke Aorta carotis communis, und linke Arteria subclavia) besteht. Proximal des Abgangs des Truncus brachiocephalicus wird nun der zweite, Stentfreie Gefäßprothesenabschnitt freigesetzt und kann mit dem proximalen Aortenbogen vernäht werden. Dieses Verfahren hat den Vorteil, dass dann, wenn zuvor bspw. bereits der aufsteigende Anteil der Aorta ascendens ersetzt worden war, diese blutungsstillende Naht simultan die Gefäßprothese mit fassen.

Die erfindungsgemäße Gefäßprothese bietet daher den Vorteil, dass ein im Bereich der Aorta ascendens vorliegender Einriss oder ein Aneurysma durch Resektion und konventionellen prothetischen Einsatz - vermittelt durch den zweiten Stentgraftabschnitt - wie bisher beseitigt werden kann, und simultan eventuell verbleibende Einrisse der Intima in der proximalen Aorta descendens oder im Aortenbogen sicher stabilisieren werden können, und zwar ohne Rupturgefahr im Langzeitverlauf. Dies kann insbesondere auch dadurch erreicht werden, dass die Gefäßprothese bspw. 10% bis 20% größer bzw. länger als jeweils notwendig ausgebildet ist (sog. "Oversizing").

Damit kann insbesondere im Falle einer Dissektionserkrankung oder komplexen aneurysmatischen Erkrankung der thorakalen Aorta der operative und zeitliche Aufwand gegenüber bisher im Stand der Technik angewandten Operationen und Systemen bei einem vergleichbaren Ergebnis gedrittelt, und damit das Risiko des Eingriffs signifikant gesenkt werden.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung einer Ausführungsform einer erfindungsgemäßen Gefäßprothese (nicht maßstabsgetreu), im nichteingeführten, aber expandierten Zustand;
- Fig. 2: eine schematische Darstellung einer weiteren Ausführungsform einer erfindungsgemäßen Gefäßprothese (nicht maßstabsgetreu), ebenfalls im nicht-eingeführten, aber expandierten Zustand; und
- Fig. 3: eine schematische Darstellung der in Fig. 1 gezeigten Ausführungsform, positioniert und freigesetzt im Aortenbogen.

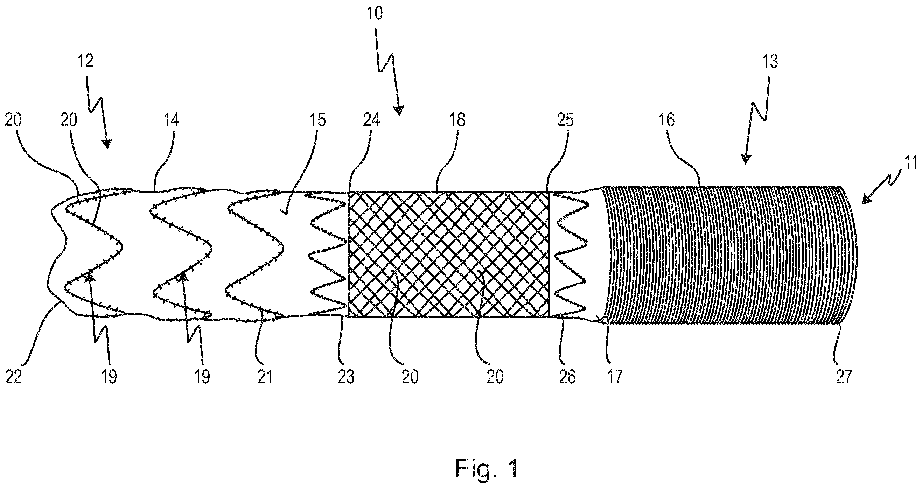

In Fig. 1 ist mit 10 insgesamt eine erfindungsgemäße Gefäßprothese gezeigt, mit einem hohlzylindrischen Körper 11, sowie mit einem ersten, distalen Ende 12, sowie einem zweiten proximalen Ende 13. An ihrem distalen Ende 12 weist sie einen ersten Gefäßprothesenabschnitt 14 auf, sowie an ihrem proximalen Ende einen zweiten Gefäßprothesenabschnitt 16. Zwischen dem ersten Gefäßprothesenabschnitt 14 und dem zweiten Gefäßprothesenabschnitt 16 befindet sich mittig ein Stentabschnitt 18 der Gefäßprothese, der frei von Prothesenmaterial ist und offene Zellen bzw. Poren 20 aufweist, durch die Blut in die abgehenden Gefäße hindurch gelangen kann.

Der erste Gefäßprothesenabschnitt 14 und der zweite Gefäßprothesenabschnitt 16 weisen beide ein Prothesenmaterial 15 bzw. 17 auf. Bei der in Fig. 1 gezeigten Gefäßprothese weist der erste Gefäßprothesenabschnitt 14 ferner hintereinander angeordnete Ringe 19 aus mäanderförmig umlaufenden Stützen 20 auf, die untereinander nur über das Prothesenmaterial 15 verbunden sind, an welches sie bspw. durch Nähte 21 angenäht sind. Die mäanderförmig umlaufenden Stützen 20 bzw. Ringe 19 bilden zusammen einen hohlzylindrischen bzw. röhrenförmigen Körper. Die Ringe 19 sind vorzugsweise aus Nitinol. Die in Fig. 1 gezeigte Ausführungsform der erfindungsgemäßen Gefäßprothese 10 weist in ihrem Gefäßprothesenabschnitt 14 vier Ringe 19 auf, wobei dem Fachmann klar ist, dass die Zahl der Ringe je nach Gefäßgegebenheit, -anforderung und Patient unterschiedlich sein kann.

Der zweite Gefäßprothesenabschnitt 16 weist in dem in Fig. 1 gezeigten Ausführungsbeispiel keine Ringe oder sonstige Stentelemente auf, sondern besteht nur aus Prothesenmaterial 17, das derart geformt ist, dass es ebenfalls einen hohlzylindrischen Körper bildet. Dieser Abschnitt wird auch als "Cuff " bezeichnet. Der erste Gefäßprothesenabschnitt 14 stellt damit einen distalen Gefäßprothesenabschnitt dar, der Stentabschnitt 18 den mittleren Gefäßprothesenabschnitt und der zweite Gefäßprothesenabschnitt 16 den proximalen Gefäßprothesenabschnit.t

Das Prothesenmaterial 15 und 17 ist dabei vorzugsweise aus einem blutdichten Material, vorzugsweise Polyestergewebe.

Der Stentabschnitt 18 der Gefäßprothese 10 kann ein Drahtgeflecht aus miteinander verflochtenen Nitinol-Drähten sein, oder aber ein Laser-geschnittener Nitinol-Stent. In beiden Fällen werden Öffnungen oder Fenster oder Maschen 20 gebildet, durch die Blut aus dem Gefäß und der Gefäßprothese hindurch dringen kann, und in umliegende und abgehende Gefäße abfließen kann.

In Fig. 1 sind ferner die Enden der einzelnen Abschnitte 14, 16 und 18 der Gefäßprothese näher bezeichnet: So weist der erste Gefäßprothesenabschnitt 14 ein distales Ende 22 auf, und grenzt mit seinem proximalen Ende 23 an das distale Ende 24 des Stentabschnitts 18; dessen proximales Ende 25 wiederum grenzt an das distale Ende 26 des zweiten Gefäßprothesenabschnitts 16, welcher mit seinem proximalen Ende 27 das äußerste proximale Ende der Gefäßprothese darstellt.

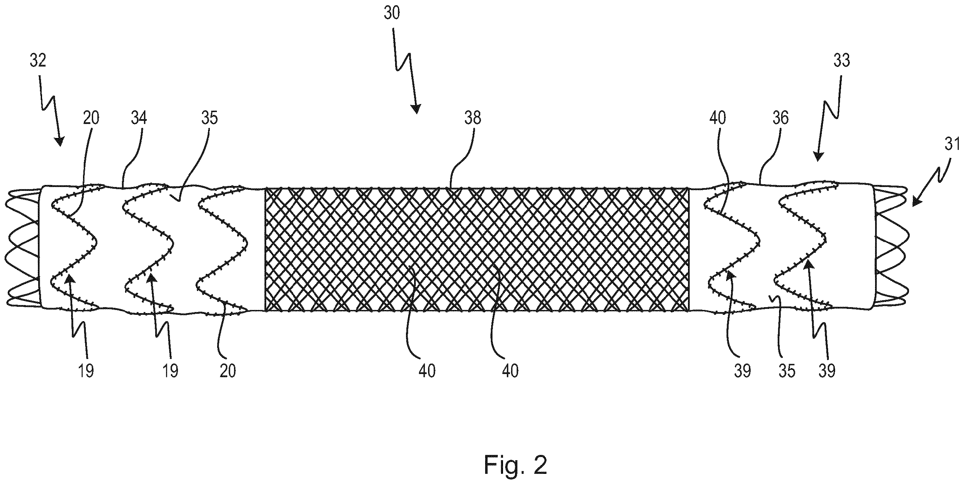

In Fig. 2 ist eine weitere Ausführungsform einer erfindungsgemäßen Gefäßprothese 30 gezeigt, wobei hier gleiche Merkmale wie die der Gefäßprothese 10 in Fig. 1 mit gleichen Bezugszeichen versehen sind. Auch die Gefäßprothese 30 weist einen hohlzylindrischen Körper 31 sowie ein distales Ende 32 und ein proximales Ende 33 auf, sowie einen ersten Gefäßprothesenabschnitt 34 und einen zweiten Gefäßprothesenabschnitt 36. Der erste Gefäßprothesenabschnitt 34 weist - wie der erste Stentgraftabschnitt 14 der in Fig. 1 gezeigten Ausführungsform 10 - hintereinander angeordnete Ringe 19 aus mäanderförmig umlaufenden Stützen 20 auf, die über ein Prothesenmaterial 35 miteinander verbunden sind. Ferner ist mit 38 ein Stentabschnitt dargestellt, der frei von Prothesenmaterial ist und offene Zellen bzw. Öffnungen 40 aufweist.

Die in Fig. 2 gezeigte Ausführungsform 30 besitzt einen zweiten Gefäßprothesenabschnitt 36, der in dieser Ausführungsform wie der erste Gefäßprothesenabschnitt 34 hintereinander angeordnete Ringe 19 aus mäanderförmig umlaufenden Stützen 20 aufweist und mit einem die Ringe 19 verbindenden Prothesenmaterial 35 bedeckt ist. Wie in Fig. 1 stellt der erste Gefäßprothesenabschnitt 34 einen distalen Gefäßprothesenabschnitt dar, der Stentabschnitt 38 den mittleren Gefäßprothesenabschnitt und der zweite Gefäßprothesenabschnitt 36 den proximalen Gefäßprothesenabschnitt.

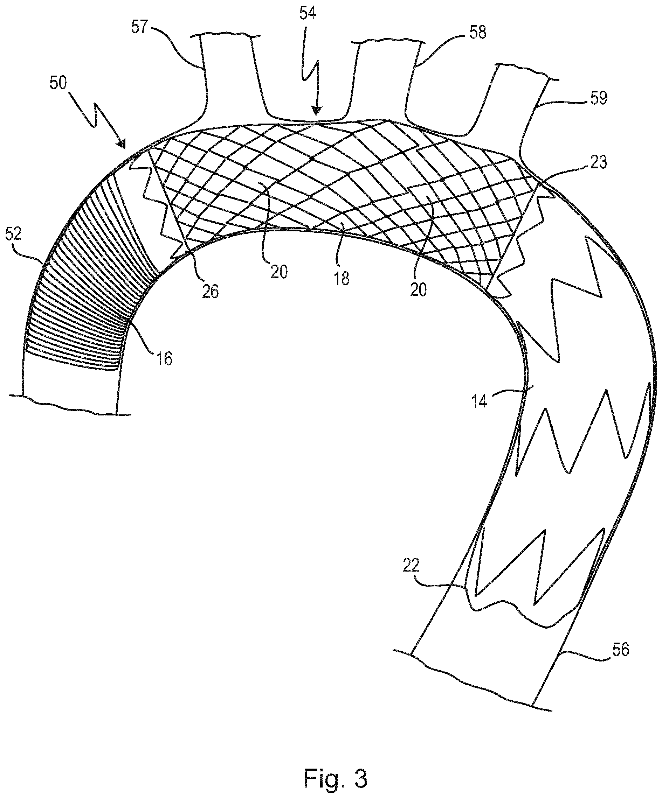

Fig. 3 zeigt schließlich die in Fig. 1 gezeigte Ausführungsform der erfindungsgemäßen Gefäßprothese in einem in eine Aorta 50 eingebrachten Zustand: In Fig. 3 ist mit 52 ein Teil der aufsteigenden - ascendierenden - Aorta bezeichnet, mit 54 der Aortenbogen und mit 56 die absteigende - descendierende - Aorta. Wie aus Fig. 3 ersichtlich, gehen im Bereich des Aortenbogens 54 drei Gefäße 57, 58, und 59 ab, nämlich der Truncus brachiocephalus 57, die Arteria carotis communis 58, und die Arteria subclavia sinistra 59.

Fig. 3 ist ferner die Lage bzw. Positionierung der in Fig. 1 dargestellten Ausführungsform der erfindungsgemäßen Gefäßprothese 10 zu entnehmen: Zu erkennen ist, dass der zweite Gefäßprothesenabschnitt 16, bzw. dessen distales Ende 26, proximal des Truncus brachiocephalus beginnt, dass ferner der mittlere und von Prothesenmaterial freie Stentabschnitt 18 im Aortenbogen 54 freigesetzt ist und über seine Zellen bzw. Öffnungen oder Maschen 20 die abgehenden Gefäße 57, 58 und 59 mit Blut versorgen kann, und dass schließlich der erste Stentgraftabschnitt 14, bzw. dessen proximales Ende 23, distal der Arteria subclavia sinistra 59 beginnt.

Zur Einführung der erfindungsgemäßen Gefäßprothese 10, 30 wird diese auf ein Einführsystem geladen (nicht gezeigt) und über eine entsprechende Hülle (nicht gezeigt) in einem komprimierten Zustand gehalten. Verfahren und Vorrichtungen zum Einführen von Gefäßprothesen sind dem Fachmann aus dem Stand der Technik geläufig. Die komprimiert gehaltene Gefäßprothese 10, 30 wird in die descendierende Aorta vorgeschoben, bis das proximale Ende 23 der Gefäßprothese 10, 30 distal der Arteria subclavia liegt. Die korrekte Platzierung kann bspw. über entsprechende, an der Gefäßprothese 10, 30 vorgesehene - bspw. röntgendichte - Marker kontrolliert werden. Nach korrekter Platzierung kann die Gefäßprothese 10, 30 nun durch Rückziehen der Hülle freigesetzt werden, wobei nach dem ersten Gefäßprothesenabschnitt 14 der von Prothesenmaterial freie - nicht-gecoverte - Stentabschnitt 18 im Aortenbogen 54 freigesetzt wird, wobei die Öffnungen bzw. Maschen 20 so weiträumig sind, dass keine Gefährdung im Sinn einer Verlegung der Abgänge der Kopf-Hals Gefäße 57, 58 und 59 (Truncuns brachiocephalicus, linke Aorta carotis communis, linke Arteria subclavia) besteht.

Abschließend wird proximal des Abgangs des Truncus brachiocephalicus 57 wird nun der zweite Gefäßprothesenabschnitt 16 freigesetzt dun kann bspw. mit dem proximalen Aortenbogen vernäht werden. Wenn zuvor bereits der aufsteigende Anteil der Aorta ascendens 52 ersetzt worden war, wird diese blutungsstillende Naht simultan diesen Stentgraftabschnitt mit fassen.

Dem Fachmann wird klar sein, dass er die genauen Maße und räumlichen Anforderungen der einzelnen Gefäßprothesenabschnitte, mithin also des ersten und des zweiten Gefäßprothesenabschnitts 14, 16 sowie des Stentabschnitts 18 durch vorherige Untersuchung des zu behandelnden Patienten ermitteln und jeweils spezifisch umsetzen kann.

## Patentansprüche

1. Intraluminale Gefäßprothese (10; 30), die einen hohlzylindrischen Körper (11; 31) mit einem ersten Ende (12; 32) und einem zweite Ende (13; 33) aufweist, wobei die Gefäßprothese (10; 30) an ihrem ersten Ende (12; 32) einen ersten Gefäßprothesenabschnitt (14; 34) mit hintereinander angeordneten Ringen (19) aus mäanderförmig umlaufenden Stützen (20) und ein an den Ringen (19) befestigtes und diese verbindendes Prothesenmaterial (15; 35) umfasst, wobei die Gefäßprothese (10; 30) an ihrem zweiten Ende (13; 33) einen zweiten Gefäßprothesenabschnitt (16; 36) aufweist, wobei die Gefäßprothese (10; 30) ferner einen zwischen dem ersten (14; 34) und dem zweiten (16; 36) Gefäßprothesenabschnitt vorliegenden und mit diesen Gefäßprothesenabschnitten (14; 34; 16; 36) fest verbundenen Stentabschnitt (18; 38) aufweist, der von Prothesenmaterial frei ist, **dadurch gekennzeichnet dass** der zweite Gefäßprothesenabschnitt (16;36) lediglich ein Prothesenmaterial (17;35) aufweist und keine damit verbundenen, hintereinander angeordneten Ringe (39) aus mäanderförmig umlaufenden Stützen (40) aufweist.

2. Intraluminale Gefäßprothese (10; 30) nach Anspruch 1, **dadurch gekennzeichnet, dass** sie zur Implantation in eine Aorta (50), insbesondere im Bereich der aufsteigenden Hauptschlagader (52), des Aortenbogens (54) und der absteigenden Hauptschlagader (56) ausgebildet ist, wobei die Gefäßprothese (10; 30) zur Einbringung in die Aorta (50) von einem komprimierten Zustand in einen expandierten Zustand überführbar ist, und wobei der erste (14; 34) und der zweite (16; 36) GEfäßprothesenabschnitt zur Verankerung der Gefäßprothese (10; 30) in der Aorta (50) ausgebildet sind.

3. Intraluminale Gefäßprothese (10; 30) nach Anspruch 1 oder 2, **dadurch gekennzeichnet, dass** der erste Gefäßprothesenabschnitt (14; 34) und der Stentabschnitt (18; 38) aus einem selbst-expandierenden Material sind oder ein solches aufweisen.

4. Intraluminale Gefäßprothese (10; 30) nach einem der Ansprüche 1 bis 3, **dadurch gekennzeichnet, dass** der von Prothesenmaterial freie Stentabschnitt (18; 38) im expandierten Zustand im Bereich des Aortenbogens (54) freisetzbar ist.

5. Intraluminale Gefäßprothese (10; 30) nach einem der Ansprüche 1 bis 4, **dadurch gekennzeichnet, dass** der erste Gefäßprothesenabschnitt (14; 34) zwischen zwei und fünf hintereinander angeordnete Ringe (19) aus mäanderförmig umlaufenden Stützen (20) aufweist.

6. Intraluminale Gefäßprothese (10; 30) nach einem der Ansprüche 1 bis 5, **dadurch gekennzeichnet, dass** der erste Gefäßprothesenabschnitt (14; 34) drei hintereinander angeordnete Ringe (19) aus mäanderförmig umlaufenden Stützen (20) aufweist, die über das Prothesenmaterial (15) miteinander verbunden sind.

7. Intraluminale Gefäßprothese (10; 30) nach einem der Ansprüche 1 bis 6, **dadurch gekennzeichnet, dass** der von Prothesenmaterial freie Stentabschnitt (18; 38) ein geflochtenes oder gezwirbeltes Drahtgeflecht aufweist.

8. Intraluminale Gefäßprothese (10; 30) nach einem der Ansprüche 1 bis 6, **dadurch gekennzeichnet, dass** der von Prothesenmaterial freie Stentabschnitt (18; 38) ein Laser-geschnittenes Rohr ist.

9. Intraluminale Gefäßprothese nach einem der Ansprüche 1 bis 8 zur Verwendung in der Behandlung von Dissektionserkrankungen oder aneurysmatischen Erkrankungen der thorakalen Aorta.

## Claims

1. An intraluminal vascular prosthesis (10; 30), having a hollow cylindrical body (11; 31) with a first end (12; 32) and a second end (13; 33), wherein the vascular prosthesis (10; 30) comprises, at its first end (12; 32), a first vascular prosthesis portion (14; 34), with successive rings (19) of meandering supports (20), and a prosthesis material (15; 35) secured on the rings (19) and connecting them, wherein the vascular prosthesis (10; 30) has, at its second end (13; 33), a second vascular prosthesis portion (16; 36), wherein the vascular prosthesis (10; 30) moreover has a stent portion (18; 38) which is provided between the first vascular prosthesis portion (14; 34) and the second vascular prosthesis portion (16; 36) and which is fixedly connected to said vascular prosthesis portions (14; 34; 16; 36), said stent portion (18; 38) being free of prosthesis material, **characterized in that** the second vascular prosthesis portion (16; 36) has only a prosthesis material (17; 35) and has no successive rings (39) of meandering supports (40) connected to the prosthesis material (17; 35).

2. The intraluminal vascular prosthesis (10; 30) as claimed in claim 1, **characterized in that** it is designed for implantation in an aorta (50), particularly in the area of the ascending aorta (52), the aortic arch (54) and the descending aorta (56), wherein the vascular prosthesis (10; 30) for introduction into the aorta (50) can be transferred from a compressed state to an expanded state, and wherein the first vascular prosthesis portion (14; 34) and the second vascular prosthesis portion (16; 36) are designed for anchoring the vascular prosthesis (10; 30) in the aorta (50).

3. The intraluminal vascular prosthesis (10; 30) as claimed in claim 1 or 2, **characterized in that** the first vascular prosthesis portion (14; 34) and the stent portion (18; 38) are made from a self-expanding material or comprise such a material.

4. The intraluminal vascular prosthesis (10; 30) as claimed in one of claims 1 through 3, **characterized in that** the stent portion (18; 38) free of prosthesis material can be released in the expanded state in the area of the aortic arch (54).

5. The intraluminal vascular prosthesis (10; 30) as claimed in one of claims 1 through 4, **characterized in that** the first vascular prosthesis portion (14; 34) has between two and five rings (19) of successive meandering supports (20).

6. The intraluminal vascular prosthesis (10; 30) as claimed in one of claims 1 through 5, **characterized in that** the first vascular prosthesis portion (14; 34) has three successive rings (19) of meandering supports (20), which are connected to one another by the prosthesis material (15).

7. The intraluminal vascular prosthesis (10; 30) as claimed in one of claims 1 through 6, **characterized in that** the stent portion (18; 38) free of prosthesis material has a braided or twisted wire braid.

8. The intraluminal vascular prosthesis (10; 30) as claimed in one of claims 1 through 6, **characterized in that** the stent portion (18; 38) free of prosthesis material is a laser-cut tube.

9. The intraluminal vascular prosthesis as claimed in one of claims 1 through 8 for use in the treatment of dissections or aneurysms of the thoracic aorta.

## Revendications

1. Prothèse vasculaire endoluminale (10; 30), qui présente un corps cylindrique creux (11; 31) avec une première extrémité (12; 32) et une deuxième extrémité (13; 33), dans laquelle la prothèse vasculaire (10; 30) comprend à sa première extrémité (12; 32) une première partie de prothèse vasculaire (14; 34) avec des anneaux (19) disposés l'un derrière l'autre constitués de supports périphériques sinueux (20) et un matériau de prothèse (15; 35) qui est fixé aux anneaux (19) et qui les solidarise, dans laquelle la prothèse vasculaire (10; 30) présente à sa deuxième extrémité (13; 33) une deuxième partie de prothèse vasculaire (16; 36), dans laquelle la prothèse vasculaire (10; 30) présente en outre une partie de stent (18; 38) placée entre la première (14; 34) et la deuxième (16; 36) partie de prothèse vasculaire et solidement reliée à ces parties de prothèse vasculaire (14; 34; 16; 36), qui est libre de matériau de prothèse, **caractérisée en ce que** la deuxième partie de prothèse vasculaire (16; 36) présente uniquement un matériau de prothèse (17; 35) et ne présente pas d'anneaux (39) reliés à celui-ci et disposés l'un derrière l'autre en supports périphériques sinueux (40).

2. Prothèse vasculaire endoluminale (10; 30) selon la revendication 1, **caractérisée en ce qu'**elle est configurée en vue de l'implantation dans une aorte (50), en particulier dans la région de l'aorte ascendante (52), de l'arc aortique (54) et de l'aorte descendante (56), dans laquelle la prothèse vasculaire (10; 30) peut être convertie d'un état comprimé à un état expansé pour l'introduction dans l'aorte (50), et dans laquelle la première (14; 34) et la deuxième (16; 36) parties de prothèse vasculaire sont conçues pour ancrer la prothèse vasculaire (10; 30) dans l'aorte (50).

3. Prothèse vasculaire endoluminale (10; 30) selon la revendication 1 ou 2, **caractérisée en ce que** la première partie de prothèse vasculaire (14; 34) et la partie de stent (18; 38) sont constituées d'un matériau auto-expansible ou en contiennent.

4. Prothèse vasculaire endoluminale (10; 30) selon l'une quelconque des revendications 1 à 3, **caractérisée en ce que** la partie de stent (18; 38) libre de matériau de prothèse peut être libérée à l'état expansé dans la région de l'arc aortique (54).

5. Prothèse vasculaire endoluminale (10; 30) selon l'une quelconque des revendications 1 à 4, **caractérisée en ce que** la première partie de prothèse vasculaire (14; 34) présente entre deux et cinq anneaux (19) disposés l'un derrière l'autre constitués de supports périphériques sinueux (20).

6. Prothèse vasculaire endoluminale (10; 30) selon l'une quelconque des revendications 1 à 5, **caractérisée en ce que** la première partie de prothèse vasculaire (14; 34) présente trois anneaux (19) disposés l'un derrière l'autre constitués de supports périphériques sinueux (20), qui sont reliés l'un à l'autre par le matériau de prothèse (15).

7. Prothèse vasculaire endoluminale (10; 30) selon l'une quelconque des revendications 1 à 6, **caractérisée en ce que** la partie de stent (18; 38) libre de matériau de prothèse présente un treillis de fils tressé ou entrelacé.

8. Prothèse vasculaire endoluminale (10; 30) selon l'une quelconque des revendications 1 à 6, **caractérisée en ce que** la partie de stent (18; 38) libre de matériau de prothèse est un tube coupé au laser.

9. Prothèse vasculaire endoluminale (10; 30) selon l'une quelconque des revendications 1 à 8 destinée à être utilisée dans le traitement de maladies de dissection ou de maladies anévrismatiques de l'aorte thoracique.
